# EUROPÄISCHE PATENTSCHRIFT

(11) **EP 0 585 265 B1**
(45) Veröffentlichungstag und Bekanntmachung des Hinweises auf die Patenterteilung: **29.05.1996**
(21) Anmeldenummer: 92909434.0
(22) Anmeldetag: 29.04.1992
(51) Int. Cl.: C07C 41/03, C07C 67/31, C07C 303/42, C07C 69/708

(54) **VERFAHREN ZUR HERSTELLUNG VON EPOXID-RINGÖFFNUNGSPRODUKTEN MIT EINEM DEFINIERTEN REST-EPOXIDSAUERSTOFFGEHALT---------------------**
PROCESS FOR PRODUCING EPOXY OPEN RING PRODUCTS WITH A DEFINED RESIDUAL EPOXY OXYGEN CONTENT
PROCEDE DE FABRICATION DE PRODUITS A OUVERTURE DE CYCLE EPOXY AYANT UNE TENEUR DETERMINEE EN OXYGENE EPOXY RESIDUEL

(30) Priorität: 08.05.1991 DE 4115146
(43) Veröffentlichungstag der Anmeldung: 09.03.1994
(73) Patentinhaber: Henkel Kommanditgesellschaft auf Aktien, 40191 Düsseldorf (DE)
(72) Erfinder: DAUTE, Peter, D-4300 Essen 1 (DE); GRÜTZMACHER, Roland, D-5603 Wülfrath (DE); MERTSCHEIT, Nicole, D-4010 Hilden (DE)
(86) Internationale Anmeldenummer: EP9200932
(87) Internationale Veröffentlichungsnummer: WO9219577

(56) Entgegenhaltungen:
- EP-A- 0 111 626
- EP-A- 0 257 332
- EP-A- 0 340 587
- EP-A- 0 361 080
- DE-B- 1 261 844
- US-A- 2 372 615

## Beschreibung

Die Erfindung betrifft Epoxid-Ringöffnungsprodukte mit einem definierten Rest-Epoxidsauerstoffgehalt sowie ein Verfahren zu ihrer Herstellung durch Umsetzung von Epoxiden mit Nucleophilen in Gegenwart mittelstarker Säuren.

Langkettige Polyolverbindungen mit zwei oder mehr freien Hydroxylgruppen und einem Anteil an Epoxidgruppen stellen begehrte Rohstoffe für die Herstellung von Polymeren, beispielsweise Polyestern, Alkydharzen oder Polyurethanschäumen dar.

Zu ihrer Herstellung geht man vorzugsweise von Olefinen oder ungesättigten Fettstoffen aus, die im ersten Schritt epoxidiert werden. Im zweiten Schritt werden die gebildeten Epoxide einer Ringöffnung mit Nucleophilen, beispielsweise Wasser oder Alkoholen, unterworfen, wobei Stoffe mit vicinal angeordneten Substituenten entstehen, von denen einer eine Hydroxylgruppe ist. Diese Öffnung des Oxiranringes ist Gegenstand einer Vielzahl von Veröffentlichungen.

So beschreibt die US-amerikanische Patentschrift **US 4 057 589** beispielsweise ein Verfahren zur Herstellung von Tetrolen durch Umsetzung von ungesättigten Diolen mit Peressigsäure und nachfolgente Hydrolyse der gebildeten Epoxide bei Temperaturen von mindestens 120 °C.

Gegenstand der Europäischen Patentanmeldung EP 0 127 810 A1 ist die schwefelsäure-katalysierte Ringöffnung von Epoxiden ungesättigter Fettsäureester mit Alkoholen sowie deren nachfolgende Verseifung.

In der Deutschen Offenlegungsschrift DE 3 246 612 A1 wird ein Verfahren zur Herstellung modifizierter Triglyceride beschrieben, bei dem man epoxidierte Fette oder Öle in Gegenwart von Schwefelsäure, Phosphorsäure oder Sulfonsäuren mit ein- oder mehrwertigen Alkoholen umsetzt. Die Reaktion wird durch Zerstören und/oder Entfernen des Katalysators und/oder der Alkoholkomponente nach einem Umsatz von 20 bis 80 mol.-% - bezogen auf die Epoxidgruppen - abgebrochen.

Die katalysatorfreie Druckspaltung von kurzkettigen Epoxiden mit 3 bis 8 Kohlenstoffatomen sowie Fettepoxiden mit 16 bis 22 Kohlenstoffatomen mit Wasser ist Gegenstand der Europäischen Patentanmeldungen EP 0 257 243 A2, EP 0 340 587 A2 sowie die EP 0 361 080 beschreibt die Ringöffnung von Epoxifettalkoholestern mit mono- oder polyfunktionellen Alkoholen. Die Umsetzung wird durch die Gegenwart geringer Mengen von Lewissäuren katalysiert.

Die DE 1 261 844 beschreibt ein Verfahren zur Herstellung von Etherwachsen, bei dem Glycidylether in Gegenwart von sauren Katalysatoren, wie z. B. Phosphorsäure oder Unterphosphoriger Säure mit Alkoholen einer Ringöffnung unterzogen werden. Die Druckschrift befaßt sich weder mit erfindungsgemäßen Substanzen noch mit dem erfindungsgemäßen Verfahren.

Die US-A-2 372 615 betrifft ein Verfahren zur Herstellung von aromatischen Ethern durch Ringöffnung von Styroloxid mit Alkoholen. Die Druckschrift erwähnt ausdrücklich die Notwendigkeit, daß die als Katalysatoren eingesetzten Säuren nach Abschluß der Reaktion neutralisiert werden müssen.

Die EP 0 111 626 A1 betrifft ein Verfahren zur Herstellung modifizierter Triglyceride, die sowohl Epoxidgruppen als auch Hydroxylgruppen und Ethergruppen aufweisen. In dieser Druckschrift ist ausdrücklich erwähnt, daß ein Neutralisationsmittel der Katalysatorsäure zugesetzt wird, sobald die gewünschte Epoxidzahl erreicht ist.

In der Europäischen Patentschrift EP 0 257 332 B1 wird schließlich ein Verfahren zur kontinuierlichen Herstellung von 1,2 Diolen beschrieben, bei dem man Epoxide in Gegenwart von sauren Katalysatoren einer Druckspaltung mit Wasser unterwirft.

Die Herstellung von Polyolverbindungen mit einem Rest-Epoxidsauerstoffgehalt nach den genannten Verfahren des Stands der Technik ist jedoch mit einer Reihe von Nachteilen behaftet:

So muß bei Einsatz der Druckspaltungs-Technologie beispielsweise durch Umesterungsreaktionen mit der Bildung unerwünschter Nebenprodukte gerechnet werden. Zudem kann die Ringöffnung der Epoxide nach diesem Verfahren nur mit Wasser durchgeführt werden, wenn der dafür erforderliche technische Aufwand die Rentabilität des Verfahrens nicht übersteigen soll. Über Druckspaltungsverfahren ist somit nur eine eingeschränkte Zahl von Produkten zugänglich.

Wird die Ringöffnung der Epoxidverbindungen hingegen in Anwesenheit saurer Katalysatoren durchgeführt, so muß der Rest-Epoxidsauerstoffgehalt durch Abtrennung oder Neutralisation der Katalysatorsäure bei einem bestimmten Epoxidwert oder durch Entfernen einer Reaktionskomponente, beispielsweise des Nucleophils, eingestellt werden. Dies ist mit erhöhtem technischen Aufwand verbunden.

Die Aufgabe der Erfindung bestand somit darin, ein verbessertes Verfahren zur Herstellung von Epoxid-Ringöffnungsprodukten mit einem definierten Rest-Epoxidsauerstoffgehalt zu entwickeln, das frei von den geschilderten Nachteilen ist.

Gegenstand der Erfindung ist ein Verfahren zur Herstellung von Epoxid-Ringöffnungsprodukten mit einem definierten Rest-Epoxidsauerstoffgehalt von 10 bis 80 Gew.-% (bezogen auf das Epoxid), dadurch gekennzeichnet, daß man aliphatische epoxydierte Olefine oder aliphatische epoxydierte Ester mit Nucleophilen in Gegenwart von 1 bis 4 g Phosphorsäure oder Phosphoriger Säure pro Mol Epoxid umsetzt und das Ringöffnungsprodukt ohne anschließende Neutralisation der Katalysatorsäure und ohne anschließende Abtrennung überschüssigen Nucleophils erhält.

Überraschenderweise wurde gefunden, daß bei Verwendung der genannten Säuren als Katalysatoren in der Ringöffnung von Epoxidverbindungen der Rest-Epoxidsauerstoffgehalt nur noch von der Katalysatormenge abhängig ist. Dadurch ist es möglich, bereits vor der Reaktion über die Katalysatormenge den gewünschten Epoxidwert des Produktes festzulegen. Hierdurch wird die bislang als obligatorisch erachtete Neutralisation des Katalysators sowie die Abtrennung des Nucleophils zur Einstellung des Rest-Epoxidsauerstoffgehaltes überflüssig.

Epoxidverbindungen stellen bekannte Stoffe dar und können nach an sich bekannten Verfahren durch Epoxidation ungesättigter Einsatzstoffe erhalten werden. Beispiele hierzu sind die Umsetzung von Olefinen mit Peressigsäure in Anwesenheit saurer Katalysatoren **[DE 857 364]** oder mit in-situ aus Ameisensäure und Wasserstoffperoxid gebildeter Perameisensäure **[US 2 485 160]**. Für die Durchführung des erfindungsgemäßen Verfahrens ist Voraussetzung, daß in den Epoxidverbindungen ein substantieller Anteil, beispielsweise 2 bis 10, vorzugsweise 4 bis 8,5 Gew.-% Epoxidsauerstoff vorhanden ist. Dies schließt mit ein, daß im Sinne des erfindungsgemäßen Verfahrens nicht nur vollständig, sondern auch partiell epoxidierte Stoffe eingesetzt werden können.

### Unter Epoxidverbindungen sollen im folgenden verstanden werden:

### al) Epoxide von Olefinen der Formel (I),

**R¹-CH=CH-R** (I)

in der R¹ für einen linearen oder verzweigten aliphatischen Kohlenwasserstoffrest mit 1 bis 18 Kohlenstoffatomen und R für Wasserstoff oder einen linearen oder verzweigten Kohlenwasserstoffrest mit 1 bis 8 Kohlenstoffatomen steht. Typische Beispiele sind die Epoxide von Octen-1, Decen-1, Dodecen-1, Tetradecen-1, Octadecen-1 oder Octadecen-9. Bevorzugt sind Epoxide von Olefinen der Formel (I), in der die Summe von R¹ und R für Zahlen von 8 bis 16 steht.

### a2) Epoxide von Estern der Formel (II),

**R³CO-OR⁴** (II)

in der R³CO für einen aliphatischen Acylrest mit 16 bis 24 Kohlenstoffatomen und 1 bis 5 Doppelbindungen und R⁴ für einen linearen oder verzweigten Alkylrest mit mit 1 bis 4 Kohlenstoffatomen steht. Typische Beispiele sind die Epoxide von Palmitoleylsäuremethylester, Ölsäuremethylester, Elaidinsäuremethylester, Petroselinsäuremethylester, Linolsäuremethylester oder Erucasäuremethylester. Bevorzugt sind Epoxide von Estern der Formel (II), in der R³CO für einen aliphatischen Kohlenwasserstoffrest mit 18 bis 22 Kohlenstoffatomen und 1 oder 2 Doppelbindungen und R⁴ für eine Methylgruppe steht.

### a3) Epoxide von Estern der Formel (III),

**R⁵CO-OR⁶** (III)

in der R⁵CO für einen aliphatischen Acylrest mit 1 bis 24 Kohlenstoffatomen und 0 oder 1 bis 5 Doppelbindungen und R⁶ für einen linearen oder verzweigten aliphatischen Kohlenwasserstoffrest mit 16 bis 24 Kohlenstoffatomen und 1 bis 5 Doppelbindungen steht. Typische Beispiele sind Epoxide von Essigsäureoleylester, Ölsäureoleylester oder Erucasäureoleylester. Bevorzugt sind Epoxide von Estern der Formel **(III)**, in der R⁵CO für einen aliphatischen Acylrest mit 18 bis 22 Kohlenstoffatomen und 1 oder 2 Doppelbindungen und R⁶ für einen aliphatischen Kohlenwasserstoffrest mit 16 bis 22 Kohlenstoffatomen und 1 oder 2 Doppelbindungen steht.

### a4) Epoxide von Glycerinfettsäureestern der Formel (IV),

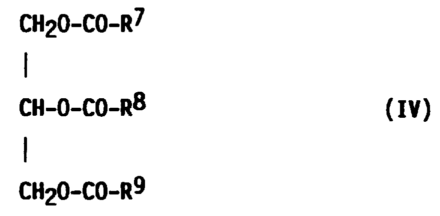

in der R⁷CO für einen linearen oder verzweigten aliphatischen Acylrest mit 16 bis 24 Kohlenstoffatomen und 1 bis 5 Doppelbindungen und R⁸CO und R⁹CO unabhängig voneinander für einen linearen oder verzweigten aliphatischen Acylrest mit 6 bis 22 Kohlenstoffatomen und 0 oder 1 bis 5 Doppelbindungen und deren Gemische steht. Typische Beispiele sind Epoxide von Erdnußöl, Korianderöl, Baumwollsaatöl, Olivenöl, Leinöl, Rindertalg, Fischöl oder insbesondere Sojaöl. Bevorzugt ist der Einsatz von Epoxiden von Glycerinfettsäureestern der Formel **(IV),** in der R⁷CO, R⁸CO und R⁹CO unabhängig voneinander für aliphatische Acylreste mit 18 bis 22 Kohlenstoffatomen und überwiegend 1 oder 2 Doppelbindungen stehen.

Als Nucleophile, die für die Ringöffnung der Epoxidverbindungen benötigt werden, kommen die folgenden Verbindungen in Betracht:
b1) Wasser
b2) Alkohole der Formel **(V),**

   **R¹⁰OH** (V)

   in der R¹⁰ für lineare oder verzweigte aliphatische Kohlenwasserstoffreste mit 1 bis 22 Kohlenstoffatomen und 0 oder 1 bis 3 Doppelbindungen steht. Typische Beispiele sind Methanol, Ethanol, Propanol-1, Propanol-2, n-Butanol, Pentanol, Hexanol, Octanol, Decanol, Laurylalkohol, Myristylalkohol, Cetylalkohol, Stearylalkohol, Oleylalkohol, Behenylalkohol oder Erucylalkohol. Bevorzugt ist der Einsatz von Methanol und Ethanol.
b3) Mehrwertige Alkohole, ausgewählt aus der Gruppe, die von Ethylenglycol, Diethylenglycol, Polyethylenglycole im Molgewichtsbereich 300 bis 1500, Propandiol-1,2, Propandiol- 1,3, Glycerin, Trimethylolpropan, Pentaerythrit, Sorbitol und Sorbitan gebildet wird.

### b4) Fettalkoholpolyglycolether der Formel (VI),

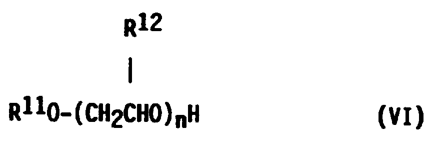

in der R¹¹ für lineare oder verzweigte aliphatische Kohlenwasserstoffreste mit 6 bis 22 Kohlenstoffatomen und 0 oder 1 bis 3 Doppelbindungen, R¹ für Wasserstoff oder eine Methylgruppe und n für Zahlen von 1 bis 30 steht. Typische Beispiele sind Anlagerungsprodukte von durchschnittlich 1 bis 30 Mol Ethylen- und/oder Propylenoxid an jeweils 1 Mol Hexanol, Octanol, Decanol, Lauryalkohol, Myristylalkohol, Cetylalkohol, Stearylalkohol, Oleylalkohol, Behenylalkohol oder Erucylalkohol. Bevorzugt ist der Einsatz von Fettalkoholpolyglycolethern der Formel **(VI),** in der R¹¹ für Alkylreste mit 6 bis 18 Kohlenstoffatomen, R¹ für Wasserstoff und n für Zahlen von 1 bis 10 steht.

Die Epoxidverbindungen und die Nucleophile können in molaren Verhältnissen von 1 : 5 bis 5 : 1, vorzugsweise 1 : 2 bis 2 : 1 eingesetzt werden. Der Einsatz der mittelstarken Säuren wird durch die Vorgabe des Rest-Epoxidsauerstoffgehaltes bestimmt. Üblicherweise kann die Menge 0,1 bis 10, vorzugsweise 1 bis 4 g pro Mol Epoxidverbindung betragen, wobei Verbindungen resultieren, die einen Restgehalt an Epoxidsauerstoff von 10 bis 80, vorzugsweise 15 bis 55 und insbesondere 20 bis 35 Gew.-% - bezogen auf die eingesetzte Epoxidverbindung - enthalten können. Im Sinne des erfindungsgemäßen Verfahrens ist die Verwendung von Phosphoriger Säure und Unterphosphoriger Säure bevorzugt.

Die partielle Ringöffnung kann in an sich bekannter Weise erfolgen, wobei es sich als vorteilhaft erwiesen hat, die Reaktion bei der Siedetemperatur des eingesetzten Nucleophils bzw. im Temperaturbereich von 100 bis 250°C durchzuführen. Wie schon zuvor ausgeführt, ist es aus Gründen der Einstellung eines speziellen Rest-Epoxidsauerstoffgehaltes nicht erforderlich, überschüssiges Nucleophil zu entfernen. Dies kann jedoch aus anderen Gründen empfehlenswert sein, wenn beispielsweise eine Veränderung der anwendungstechnischen Eigenschaften der Produkte zu befürchten ist oder ein Restgehalt des Nucleophils bei der Weiterverarbeitung stört. In diesem Fall kann sich an die Ringöffnung beispielsweise eine Destillation anschließen.

Die nach dem erfindungsgemäßen Verfahren erhältlichen Produkte eignen sich als Rohstoffe zur Herstellung von Polymeren. Sie können beispielsweise in Alkydharze sowohl über die Epoxidgruppen als auch über die Hydroxylfunktionen einkondensiert werden und stellen mehrfunktionelle Polykondensationsbausteine dar, wie sie auch für die Entwicklung von Polyurethanschäumen von Wichtigkeit sind.

Ein weiterer Gegenstand der Erfindung betrifft daher Epoxid-Ringöffnungsprodukte mit einem definierten Rest-Epoxidsauerstoffgehalt, dadurch erhältlich, daß man Epoxidverbindungen mit Nucleophilen in Gegenwart von mittelstarken Säuren aus der Gruppe, die von Phosphorsäure, Phosphoriger Säure und Unterphosphoriger Säure gebildet wird, umsetzt und gegebenenfalls die im Überschuß vorliegenden Nucleophile anschließend ohne Neutralisation abtrennt.

Die folgenden Beispiele sollen den Gegenstand der Erfindung näher erläutern, ohne ihn darauf einzuschränken.

### Beispiele

### Beispiel 1:

**Ringöffnung von Sojaölepoxid mit Methanol.** 479 g Sojaölepoxid mit einem Epoxidsauerstoffgehalt von 6,68 Gew.-% - entsprechend 2 mol Epoxid - wurden mit 128 g (4 mol) Methanol und 4,7 g 85 gew.-%iger Phosphorsäure - entsprechend 2 g pro mol Epoxid - versetzt. Die Mischung wurde über einen Zeitraum von 3 h unter Rühren bis zum Rückfluß erhitzt und anschließend auf zwei Ansätze verteilt. Ansatz 1 wurde unter Zusatz von 1,7 g Diethanolamin neutralisiert, Ansatz 2 wurde nicht neutralisiert. Beide Proben wurden im Anschluß unter vermindertem Druck bei ca. 65°C von nichtumgesetztem Methanol befreit.

Rest-Epoxidsauerstoffgehalt Ansatz 1: 4,59 Gew.-%

Rest-Epoxidsauerstoffgehalt Ansatz 2: 4,51 Gew.-%

### Beispiele 2 bis 5, Vergleichsbeispiele V1 und V2:

Analog Beispiel 1 wurden 479 g Sojaölepoxid mit einem Epoxidsauerstoffgehalt von 6,68 Gew.-% - entsprechend 2 mol Epoxid - mit 128 g (4 mol) Methanol und und jeweils 1 bzw. 1,2 g der Katalysatorsäuren A) bis E) pro mol Epoxid - versetzt. Die Mischung wurde über einen Zeitraum von 6 h unter Rühren bis zum Rückfluß erhitzt und durch regelmäßige Probenentnahme der Rest-Epoxidsauerstoffgehalt als Funktion der Zeit bestimmt.

### Eingesetzte Katalysatorsäuren:

A) Phosphorsäure (85 gew.-%ig)
B) Phosphorige Säure (100 gew.-%ig)
C) Unterphosphorige Säure (50 gew.-%ig)
D) Schwefelsäure (100 gew.-%ig)
E) Sulfobernsteinsäure (70 gew.-%ig)

Die Katalysatorsäuren A) bis C) sind erfindungsgemäß, die Säuren D) und E) dienen dem Vergleich. Die Ergebnisse sind in Tab.1 zusammengefaßt.

**Tab. 1:**

| Ringöffnung von Sojaölepoxid mit Methanol | | | | | | | | | |
|---|---|---|---|---|---|---|---|---|---|
| Bsp. | Katalysatorsäure | Menge* g/mol | Rest-Epoxidsauerstoffgehalt (%) # | | | | | | |
| | | | 0,5 | 1 | 2 | 3 | 4 | 5 | 6 |
| 2 | A | 1,0 | 4,9 | 4,6 | 4,6 | 4,6 | 4,6 | 4,6 | 4,6 |
| 3 | A | 1,2 | 5,0 | 4,6 | 4,4 | 4,4 | 4,4 | 4,4 | 4,4 |
| 4 | B | 1,0 | 4,6 | 4,3 | 4,0 | 3,9 | 3,8 | 3,8 | 3,8 |
| 5 | C | 1,0 | | 2,5 | 1,6 | 1,3 | 1,0 | 0,8 | 0,7 |
| | | | | | | | | | |
| V1 | D | 1,0 | 4,3 | 4,1 | 3,7 | 3,6 | 3,4 | 2,9 | 2,8 |
| V2 | E | 1,0 | 4,3 | 4,0 | 3,5 | 2,8 | 2,5 | 2,0 | 1,6 |

| | | | | | | | | | |
|---|---|---|---|---|---|---|---|---|---|
| *) Katalysatormenge in g pro mol Epoxid | | | | | | | | | |
| #) Rest-Epoxidsauerstoffgehalt in Gew.-% nach h | | | | | | | | | |

## Patentansprüche

1. Verfahren zur Herstellung von Epoxid-Ringöffnungsprodukten mit einem definierten Rest-Epoxidsauerstoffgehalt von 10 bis 80 Gew.-% (bezogen auf das Epoxid), dadurch gekennzeichnet, daß man aliphatische epoxydierte Olefine oder aliphatische epoxydierte Ester mit Nucleophilen in Gegenwart von 1 bis 4 g Phosphorsäure oder Phosphoriger Säure pro Mol Epoxid umsetzt und das Ringöffnungsprodukt ohne anschließende Neutralisation der Katalysatorsäure und ohne anschließende Abtrennung überschüssigen Nucleophils erhält.

2. Verfahren nach Anspruch 1, **dadurch gekennzeichnet,** daß man als Epoxidverbindungen Epoxide von Olefinen der Formel **(I),**
**R¹-CH=CH-R** (I)
einsetzt, in der R¹ für einen linearen oder verzweigten aliphatischen Kohlenwasserstoffrest mit 1 bis 18 Kohlenstoffatomen und R für Wasserstoff oder einen linearen oder verzweigten Kohlenwasserstoffrest mit 1 bis 8 Kohlenstoffatomen steht.

3. Verfahren nach Anspruch 1, **dadurch gekennzeichnet,** daß man als Epoxidverbindungen Epoxide von Estern der Formel **(II),**
**R³CO-OR⁴** (II)
einsetzt, in der R³CO für einen aliphatischen Acylrest mit 16 bis 24 Kohlenstoffatomen und 1 bis 5 Doppelbindungen und R⁴ für einen linearen oder verzweigten Alkylrest mit mit 1 bis 4 Kohlenstoffatomen steht.

4. Verfahren nach Anspruch 1, **dadurch gekennzeichnet,** daß man als Epoxidverbindungen Epoxide von Estern der Formel **(III),**
**R⁵CO-OR⁶** (III)
einsetzt, in der R⁵CO für einen aliphatischen Acylrest mit 1 bis 24 Kohlenstoffatomen und 0 oder 1 bis 5 Doppelbindungen und R⁵ für einen linearen oder verzweigten aliphatischen Kohlenwasserstoffrest mit 16 bis 24 Kohlenstoffatomen und 1 bis 5 Doppelbindungen steht.

5. Verfahren nach Anspruch 1, **dadurch gekennzeichnet,** daß man als Epoxidverbindungen Epoxide von Glycerinfettsäureestern der Formel **(IV),** 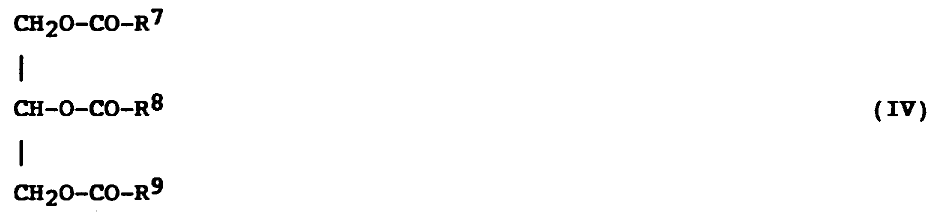 einsetzt, in der R⁷CO für einen linearen oder verzweigten aliphatischen Acylrest mit 16 bis 24 Kohlenstoffatomen und 1 bis 5 Doppelbindungen und R⁸CO und R⁹CO unabhängig voneinander für einen linearen oder verzweigten aliphatischen Acylrest mit 6 bis 24 Kohlenstoffatomen und 0 oder 1 bis 5 Doppelbindungen steht.

6. Verfahren nach mindestens einem der Ansprüche 1 bis 5, **dadurch gekennzeichnet,** daß man als Nucleophil Wasser einsetzt.

7. Verfahren nach mindestens einem der Ansprüche 1 bis 5, **dadurch gekennzeichnet,** daß man als Nucleophile Alkohole der Formel (V) einsetzt,
**R¹⁰OH** (V)
in der R¹⁰ für lineare oder verzweigte aliphatische Kohlenwasserstoffreste mit 1 bis 22 Kohlenstoffatomen und 0 oder 1 bis 3 Doppelbindungen steht.

8. Verfahren nach mindestens einem der Ansprüche 1 bis 5, **dadurch gekennzeichnet,** daß man als Nucleophile mehrwertige Alkohole einsetzt, ausgewählt aus der Gruppe, die von Ethylenglycol, Diethylenglycol, Polyethylenglycole im Molgewichtsbereich 300 bis 1500, Propandiol-1,2, Propandiol-1,3, Glycerin, Trimethylolpropan, Pentaerythrit, Sorbitol und Sorbitan gebildet wird.

9. Verfahren nach mindestens einem der Ansprüche 1 bis 5, **dadurch gekennzeichnet,** daß man als Nucleophile Fettalkoholpolyglycolether der Formel **(VI)** einsetzt, 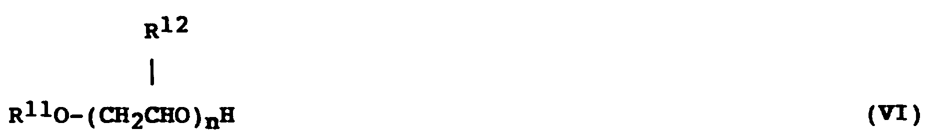 in der R¹¹ für lineare oder verzweigte aliphatische Kohlenwasserstoffreste mit 6 bis 22 Kohlenstoffatomen und 0 oder 1 bis 3 Doppelbindungen, R¹ für Wasserstoff oder eine Methylgruppe und n für Zahlen von 1 bis 30 steht.

10. Verfahren nach mindestens einem der Ansprüche 1 bis 9, **dadurch gekennzeichnet,** daß man die Epoxidverbindungen und die Nucleophile in molaren Verhältnissen von 1 : 5 bis 5 : 1 einsetzt.

11. Verfahren nach mindestens einem der Ansprüche 1 bis 11, **dadurch gekennzeichnet,** daß man die partielle Ringöffnung bei der Siedetemperatur des eingesetzten Nucleophils bzw. im Temperaturbereich von 100 bis 250°C durchführt.

## Claims

1. A process for the production of epoxide ring opening products having a defined residual epoxide oxygen content of 10 to 80% by weight (based on the epoxide), characterized in that aliphatic epoxidized olefins or aliphatic epoxidized esters are reacted with nucleophiles in the presence of 1 to 4 g of phosphoric acid or phosphorous acid per mole of epoxide and the ring opening product is directly obtained without subsequent neutralization of the catalyst acid and without subsequent removal of excess nucleophile.

2. A process as claimed in claim 1, characterized in that epoxides of olefins corresponding to formula (I):
R¹-CH=CH-R (I)
in which R¹ is a linear or branched aliphatic hydrocarbon radical containing 1 to 18 carbon atoms and R is hydrogen or a linear or branched hydrocarbon radical containing 1 to 8 carbon atoms, are used as the epoxide compounds.

3. A process as claimed in claim 1, characterized in that epoxides of esters corresponding to formula (II):
R³CO-OR⁴ (II)
in which R³CO is an aliphatic acyl radical containing 16 to 24 carbon atoms and 1 to 5 double bonds and R⁴ is a linear or branched alkyl radical containing 1 to 4 carbon atoms, are used as the epoxide compounds.

4. A process as claimed claim 1, characterized in that epoxides of esters corresponding to formula (III):
R⁵CO-OR⁶ (III)
in which R⁵CO is an aliphatic acyl radical containing 1 to 24 carbon atoms and 0 or 1 to 5 double bonds and R⁶ is a linear or branched aliphatic hydrocarbon radical containing 16 to 24 carbon atoms and 1 to 5 double bonds, are used as the epoxide compounds.

5. A process as claimed in claim 1, characterized in that epoxides of glycerol fatty acid esters corresponding to formula (IV): 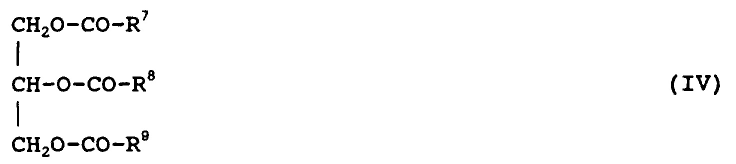 in which R⁷CO is a linear or branched aliphatic acyl radical containing 16 to 24 carbon atoms and 1 to 5 double bonds and R⁸CO and R⁹CO independently of one another represent a linear or branched aliphatic acyl radical containing 6 to 24 carbon atoms and 0 or 1 to 5 double bonds, are used as the epoxide compounds.

6. A process as claimed in at least one of claims 1 to 5, characterized in that water is used as the nucleophile.

7. A process as claimed in at least one of claims 1 to 5, characterized in that alcohols corresponding to formula (V):
R¹⁰OH (V)
in which R¹⁰ represents linear or branched aliphatic hydrocarbon radicals containing 1 to 22 carbon atoms and 0 or 1 to 3 double bonds, are used as the nucleophiles.

8. A process as claimed in at least one of claims 1 to 5, characterized in that alcohols selected from the group consisting of ethylene glycol, diethylene glycol, polyethylene glycols having a molecular weight in the range from 300 to 1500, propane-1,2-diol, propane-1,3-diol, glycerol, trimethylol propane, pentaerythritol, sorbitol and sorbitan are used as the nucleophiles.

9. A process as claimed in at least one of claims 1 to 5, characterized in that fatty alcohol polyglycol ethers corresponding to formula (VI): 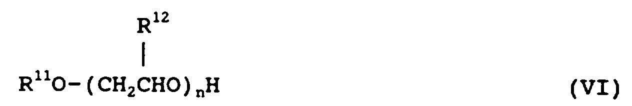 in which R¹¹ represents linear or branched aliphatic hydrocarbon radicals containing 6 to 22 carbon atoms and 0 or 1 to 3 double bonds, R¹ is hydrogen or a methyl group and n is a number of 1 to 30, are used as the nucleophiles.

10. A process as claimed in at least one of claims 1 to 9, characterized in that the epoxide compounds and the nucleophiles are used in molar ratios of 1:5 to 5:1.

11. A process as claimed in at least one of claims 1 to 10, characterized in that the partial ring opening is carried out at the boiling temperature of the nucleophile used or at a temperature in the range from 100 to 250°C.

## Revendications

1. Procédé de fabrication de produits d'ouverture du cycle époxy possédant une teneur résiduelle déterminée en oxygène de 10 à 80 % en poids (par rapport à l'époxyde), caractérisé en ce que l'on fait réagir des oléfines aliphatiques époxydées ou des esters aliphatiques époxydés avec des nucléophiles, en présence de 1 à 4 g d'acide phosphorique ou d'acide phosphoreux par mole d'époxyde, et que l'on obtient le produit d'ouverture du cycle sans neutralisation postérieure de l'acide catalyseur et sans séparation postérieure du nucléophile en excès.

2. Procédé selon la revendication 1, **caractérisé en ce que** l'on met en oeuvre comme composés époxydiques, des époxydes d'oléfines de la formule (I)
R¹-CH=CH-R (I)
dans laquelle R¹ représente un radical d'hydrocarbure aliphatique linéaire ou ramifié comportant 1 à 18 atomes de carbone et R correspond à l'hydrogène ou à un radical d'hydrocarbure linéaire ou ramifié possédant 1 à 8 atomes de carbone.

3. Procédé selon la revendication 1, **caractérisé en ce que** l'on met en oeuvre comme composés époxydiques, des esters de la formule (II),
R³CO-OR⁴ (II)
dans laquelle R³CO représente un radical acyle aliphatique comportant 16 à 24 atomes de carbone et 1 à 5 doubles liaisons, et R⁴ correspond à un radical alkyle linéaire ou ramifié possédant 1 à 4 atomes de carbone.

4. Procédé selon la revendication 1, **caractérisé en ce que** l'on met en oeuvre comme composés époxydiques, des époxydes d'esters de la formule (III),
R⁵CO-OR⁶ (III)
dans laquelle R⁵CO représente un radical acyle aliphatique comportant 1 à 24 atomes de carbone et 0 ou 1 à 5 doubles liaisons, et R⁶ correspond à un radical d'hydrocarbure aliphatique linéaire ou ramifié comportant 16 à 24 atomes de carbone et 1 à 5 doubles liaisons.

5. Procédé selon la revendication 1, **caractérisé en ce que** l'on met en oeuvre comme composés époxydiques, des époxydes d'esters de glycérine d'acides gras de la formule (IV), 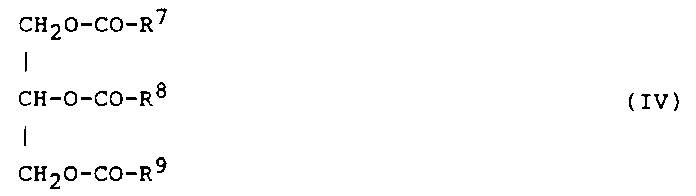 dans laquelle R⁷CO représente un radical acyle aliphatique linéaire ou ramifié comportant 16 à 24 atomes de carbone et 1 à 5 doubles liaisons, et R⁸CO et R⁹CO, correspondent indépendamment l'un de l'autre, à un radical acyle aliphatique linéaire ou ramifié comportant 6 à 24 atomes de carbone et 0 ou 1 à 5 doubles liaisons.

6. Procédé selon au moins une des revendications 1 à 5, **caractérisé en ce que** l'on met en oeuvre comme nucléophile, de l'eau.

7. Procédé selon au moins une des revendications 1 à 5, **caractérisé en ce que** l'on met en oeuvre comme nucléophiles, des alcools de la formule (V)
R¹⁰OH (V)
dans laquelle R¹⁰ représente un radical d'hydrocarbure aliphatique linéaire ou ramifié comportant 1 à 22 atomes de carbone et 0 ou 1 à 3 doubles liaisons.

8. Procédé selon au moins une des revendications 1 à 5, **caractérisé en ce que** l'on met en oeuvre comme nucléophiles, des alcools polyvalents, sélectionnés parmi le groupe formé des composés suivants: éthylèneglycol, diéthyléneglycol, polyéthylèneglycols dans le domaine de poids moléculaire de 300 à 1500, propanediol-1,2, propanediol-1,3, glycérine, triméthylolpropane, pentaérythrite, sorbitol et sorbitane.

9. Procédé selon au moins une des revendications 1 à 5, **caractérisé en ce que** l'on met en oeuvre comme nucléophiles, des polyglycoléthers d'alcools gras de la formule (VI), 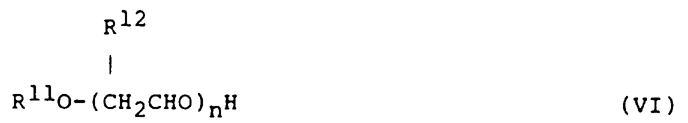 dans laquelle R¹¹ représente un radical d'hydrocarbure aliphatique linéaire ou ramifié comportant 6 à 22 atomes de carbone et 0 ou 1 à 3 doubles liaisons, R¹ correspond à l'hydrogène ou à un groupe méthyle et n représente un nombre de 1 à 30.

10. Procédé selon au moins une des revendications 1 à 9, **caractérisé en ce que** l'on met en oeuvre les composés époxydiques et les nucléophiles dans un rapport molaire de 1:5 à 5:1.

11. Procédé selon au moins une des revendications 1 à 11, **caractérisé en ce que** l'on réalise l'ouverture partielle du cycle à la température d'ébullition du nucléophile mis en oeuvre ou dans la plage de température de 100 à 250 °C.
